# EUROPEAN PATENT SPECIFICATION

(11) **EP 1 426 052 B1**
(45) Date of publication and mention of the grant of the patent: **02.09.2009**
(21) Application number: 02292963.2
(22) Date of filing: 02.12.2002
(51) Int. Cl.: A61K 31/66, A61K 31/665, A61K 38/20, A61P 37/04, A61P 31/12

(54) **Compositions comprising interleukin-2 and gamma-delta T cell activator and uses thereof**
Interleukin-2 und Gamma Delta T Zellaktivator enthaltende Zusammensetzungen und deren Verwendungen
Compositions contenant de l'interleukine-2 et un composé activant les cellules T gamma delta, et leurs utilisations

(43) Date of publication of application: 09.06.2004
(73) Proprietor: Innate Pharma, 13009 Marseille (FR)
(72) Inventor: Romagne, François, 13600 La Ciotat (FR); Sicard, Hélène, 13009 Marseille (FR); Tiollier, Jérôme, 13008 Marseille (FR)
(74) Representative: Becker, Philippe

(56) References cited:
- EP-A- 1 153 928
- WO-A-00/00158
- WO-A-00/12516
- WO-A-00/12519
- DATABASE BIOSIS [Online] BIOSCIENCES INFORMATION SERVICE, PHILADELPHIA, PA, US; 16 November 2000 (2000-11-16) KUNZMANN VOLKER ET AL: "Phase I/II trial of pamidronate and interleukin-2 (IL-2) for relapsed/refractory multiple myeloma (MM) and low-grade non-Hodgkin's lymphoma (NHL)." Database accession no. PREV200100320133 XP002239727 & BLOOD, vol. 96, no. 11 Part 1, 16 November 2000 (2000-11-16), page 733a 42nd Annual Meeting of the American Society of Hematology;San Francisco, California, USA; December 01-05, 2000 ISSN: 0006-4971
- BAIGENT GREG ET AL: "Recombinant Interleukin-2 (aldesleukin) for oncology and HIV disease and recombinant protein treatment (Fabrazyme) for Fabry's disease (No. 14 in a series of articles to promote a better understanding of the use of genetic engineering)." JOURNAL OF BIOTECHNOLOGY, vol. 95, no. 3, 2002, pages 277-282, XP002239728 23 May, 2002 ISSN: 0168-1656
- ESPINOSA E ET AL: "Y2K+1 state-of-the-art on non-peptide phosphoantigens, a novel category of immunostimulatory molecules." MICROBES AND INFECTION / INSTITUT PASTEUR. FRANCE JUL 2001, vol. 3, no. 8, July 2001 (2001-07), pages 645-654, XP002239729 ISSN: 1286-4579

## Description

The present invention relates to compositions an uses thereof for regulating an immune response in a subject, particularly a T cell response in a subject. The present invention more specifically discloses efficient methods of regulating the innate immunity in a subject, such as by regulating the activity of γδ T cells in a subject. The invention discloses that particular combinations of particular agents, such as a cytokine and a synthetic activator, can produce a remarkable expansion of γδ T cells in vivo and a remarkable increase in a subject's immune defense. The invention can be used for therapeutic purposes, to produce, regulate or facilitate an immune response in a subject. It is particularly suited to regulate a protective immune response in subjects having a cancer or infectious disease.

### 1-Introduction

T cells of the γδ type are expressed by most mammalian species. They represent 1-10% of total circulating lymphocytes in healthy adult human subjects and most non-human primates. Most human peripheral blood γδ T cells express a γδTCR heterodimer encoded by Vγ9/Vδ2 genes, some NK-lineage receptors for MHC class I and almost no CD4 nor CD8. These cells have been shown to exhibit strong, non MHC-restricted, cytolytic activity against virus-infected cells (Poccia et al, J. Leukocyte Biology, 62, 1997, p. 1-5), parasite-infected cells (Constant et al, Infection and Immunity, vol. 63, n° 12, Dec. 1995, p. 4628-4633), or tumor cells (Foumie et Bonneville, Res. Immunol., 66th FORUM IN IMMUNOLOGY, 147, p. 338-347). These cells are also physiologically amplified in the context of several unrelated infectious diseases such as tuberculosis, malaria, tularemia, colibacillosis and also by B-cell tumors (for review see Hayday, 2000).

These cells are thus viewed as potent effectors of innate immunity. Because of their potent activity against tumor cells or infected cells, γδ T cells represent a very attractive candidate for immunotherapies. Notably, since activated γδ T cells exert potent cytolytic activity and T_{H}-1 cytokine secretion, these cells represent an important resource of antiinfectious and antitumoral effectors. Accordingly, it would be highly valuable to have methods available for regulating the activity (including the expansion and/or cytolytic activity, for instance), of these cells in vivo, in a subject.

In microbes, Vγ9/Vδ2⁺ lymphocytes spontaneously recognize a structurally related set of nonpeptide antigens, referred to as natural phosphoantigens and alkylamines. In B cell tumors, the nature of antigens for the γδ T cells remains unidentified. Vγ9/Vδ2⁺ lymphocytes are also responsive to a variety of virally infected-, activated- or tumoral cell types without prior exposure. Again, in these situations, the responsible antigens remain unknown (for review see Fisch, 2000). It has been shown that, in vitro, Vγ9/Vδ2⁺ lymphocytes respond to synthetic drugs such as therapeutic aminobisphosphonates (reviewed in Espinosa, 2001), leading to their in vitro activation. Recognition of natural non-peptide antigens is mediated by the γδ TCR, through amino acid residues located on both Vγ9- and Vδ2- CDR3 regions. Although neither processing nor presentation by CD1 or MHC molecules is involved, Vγ9/Vδ2⁺ lymphocyte activation by non-peptide antigens appears to require cell-to-cell contact [Lang, 1995 ; Morita, 1995 ; Miyagawa, 2001 , Rojas, 2002]. The first set of clinical evidence for in vivo expansion of human Vγ9/Vδ2⁺ lymphocytes induced by phosphoantigen agonists came from the finding of increases of circulating γδ T cells within one to three weeks in humans adults with multiple myeloma after therapeutic intravenous injection of 60-90 mg of pamidronate [Kunzmann, 1999]. However, the actual mode of action of pamidronate is unclear and might involve an indirect effect on accessory cells [Miyagawa, 2001]. Furthermore, no appropriate conditions were disclosed to allow optimized expansion of γδ T cells using such compound. Accordingly, up to now, no efficient method has been reported to selectively activate γδ T cells in vivo, in a subject, under conditions suitable for therapy.

The present invention now proposes novel approaches and strategies for efficient regulation of γδ T cells in vivo, in a human subject.

The present invention now discloses particular compositions and uses thereof that can be used to efficiently regulate the activity of γδ T cells in vivo, in a subject. These compositions and methods are particularly suited for immuno-therapy in a subject, particularly in a subject having a cancer or an infectious disease.

The present invention more specifically relates to the use of a synthetic γδT activator and an interleukin-2 polypeptide, for the manufacture of a pharmaceutical composition for the treatment of cancer, an infections disease, an anto immune disease or an allergic disease in a human subject. The synthetic γδT activator and interleukin-2 polypeptide are administered separately. The interleukin-2 polypeptide is administered at low doses, typically over a period of time comprised between 1 and 10 days. The synthetic γδT activator is administered in a single dose comprised between 2 and 60 mg/kg at the beginning of the treatment.

Within the context of the present invention, the expression "regulating the activity of γδ T cells" designates causing or favoring an increase in the number and/or biological activity of such cells in a subject. Regulating thus includes modulating (e.g., stimulating) expansion of such cells in a subject and/or, for instance, triggering of cytokine secretion (e.g., TNFα or IFNγ). As indicated, γδ T cells normally represent between about 1-10% of total circulating lymphocytes in a healthy adult human subject. The present invention can be used to significantly increase the γδ T cells compartment in a subject, particularly to reach 30-90% of total circulating lymphocytes, typically 40-90%, more preferably from 50-90%. In typical embodiments, the invention allows the selective expansion of γδ T cells in a subject, to reach 60-90% of total circulating lymphocytes, preferably 70-90%, more preferably from 80-90%. Regulating also includes, in addition or in the alternative, modulating the biological activity of γδ T cells in a subject, particularly their cytolytic activity or their cytokine-secretion activity. The invention defines novel conditions and strategies for increasing the biological activity of γδ T cells towards target cells.

In the above methods and uses, the subject is a human subject, subject having a cancer, an infectious disease, an autoimmune disease or an allergic disease. The invention is indeed suitable to treat these conditions caused by or associated with the presence of pathological cells which are sensitive to γδ T cell lysis.

The invention is particularly suited to stimulate the anti-tumor immunity of a subject having a solid or hematopoietic tumor, such as a lymphoma, bladder cancer, multiple myeloma, renal cell carcinoma, etc.

The invention is also suitable to stimulate an anti-viral immune response in a subject having an infection by a virus selected from HIV, CMV, EBV, Influenza virus, HCV, HBV, etc.

The invention is also suitable to stimulate an immune response in a subject having an infection by a pathogen causing tuberculosis, malaria, tularemia, colibacillosis, etc.

The invention is also suitable to treat (e.g., to stimulate an immune response in) a subject having an auto-immune disease, such as diabetes, multiple sclerosis, rheumatoid arthritis, etc. or a subject having an allergic disease, including asthma, airway hyper-responsiveness, etc.

The present invention more specifically relates to the use of a synthetic γδT activator and an interleukin-2 polypeptide, for the manufacture of a pharmaceutical composition for treating cancer in a human subject, wherein said synthetic γδT activator and interleukin-2 polypeptide are administered separately to the subject. The interleukin-2 polypeptide is administered at low doses, typically over a period of time comprised between 1 and 10 days, and the synthetic γδT activator is administered in a single dose comprised between 2 and 60 mg/kg at the beginning of the treatment.

In an other specific embodiment, the present invention relates to the use of a synthetic γδT activator and an interleukin-2 polypeptide, for the manufacture of a pharmaceutical composition for treating an infectious disease in a human subject, wherein said synthetic γδT activator and interleukin-2 polypeptide are administered separately to the subject. The interleukin-2 polypeptide is administered at low doses, typically over a period of time comprised between 1 and 10 days, and the synthetic γδT activator is administered in a single dose at the beginning of the treatment at a dose comprised between 2 and 60 mg/kg.

In a further embodiment, the present invention relates to the use of a synthetic γδT activator and an interleukin-2 polypeptide, for the manufacture of a pharmaceutical composition for treating an auto-immune disease in human subject, wherein said synthetic γδT activator and interleukin-2 polypeptide are administered separately to the subject. The interleukin-2 polypeptide is administered at low doses, typically over a period of time comprised between 1 and 10 days, and the synthetic γδT activator is administered in a single dose at the beginning of the treatment, at a dose comprised between 2 and 60 mg/kg.

The above treatments may be used alone or in combination with other active agents or treatments. For instance, for the treatment of tumors, the invention may be used in combination with other anti-tumor agents or treatments, such as chemotherapy, radiotherapy or gene therapy.

The invention also relates to a product comprising a synthetic γδT activator and an interleukin-2 polypeptide, for separate use, for regulating the activity of γδ T cells in a mammalian subject, as defined in claims 13.

### Synthetic γδT lymphocytes activators

An advantageous aspect of this invention resides in the use of a synthetic γδT lymphocytes activating compound. Indeed, the invention shows that a potent and targeted expansion and activation of γδT cells can be obtained in vivo by trigerring one single metabolic pathway, using defined activating compounds following a particular administration schedule.

The term synthetic γδT lymphocytes activating compound designates a molecule artificially produced, which can activate γδT lymphocytes. It is more preferably a ligand of the T receptor of γδT lymphocytes. The activator may by of various nature, such as a peptide, lipid, small molecule, etc. It may be a purified or otherwise artificially produced (e.g., by chemical synthesis) endogenous ligand, or a fragment or derivative thereof, or an antibody having substantially the same antigenic specificity. The activator is most preferably a synthetic chemical compound capable of selectively activating Vγ9Vδ2 T lymphocytes. Selective activation of Vγ9Vδ2 T lymphocytes indicates that the compound has a selective action towards specific cell populations, and essentially does not activate other T cell sub-types, such as Vδ1 T cells. Such selectivity, as disclosed in the present application, suggests that preferred compounds can bind to the TCR bearing Vγ9 and Vδ2 components, to cause a selective or targeted activation of the proliferation or biological activity of Vγ9Vδ2 T lymphocytes.

Various distinct synthetic γδT lymphocytes activating compounds may be used, such as synthetic phosphoantigens agonists, alkylamines, (amino)biphosphonates, etc. Such compounds have been disclosed for instance in Espinosa et al (Microbes and Infection 3 (2001) 645), incorporated therein by reference. Particular classes of compounds for use in the present invention comprise phosphohalohydrins (PHD), as disclosed in WO00/12516, phosphoepoxydes (PED), as disclosed in WO00/12519, and biphosphonates, as disclosed by Kunzmann et al (Blood 96 (2000) 384).

Specific, preferred synthetic γδT lymphocytes activating compounds for use in the present invention are phosphohalohydrins and phosphoepoxydes of general formula (I) et (II), respectively : 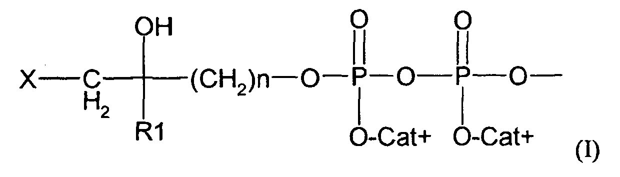 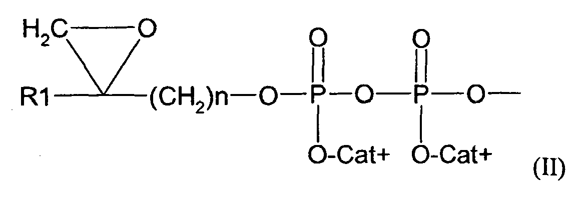 in which X is an halogen (preferably selected from I, Br and Cl), R1 is a methyl or ethyl group, Cat+ represents one (or several, identical or different) organic or mineral cation(s) (including the proton), and n is an integer comprised between 2 et 20. These compounds may be produced according to various techniques known per se in the art, some of which being disclosed in details in WO00/12516 and WO00/12519. Particular compounds are di- or tri-phosphates of formula (I) or (II) above.

In a preferred variant of this invention, a PED or a PHD compound is being used. Specific examples of such compounds include:
3-(bromomethyl)-3-butanol-1-yl-diphosphate (BrHPP)
3-(iodomethyl)-3-butanol-1-yl-diphosphate (IHPP)
3-(chloromethyl)-3-butanol-1-yl-diphosphate (CIHPP)
3-(bromomethyl)-3-butanol-1-yl-triphosphate (BrHPPP)
3-(iodomethyl)-3-butanol-1-yl-triphosphate (IHPPP)
α,γ-di-[3-(bromomethyl)-3-butanol-1-yl]-triphosphate (diBrHTP)
α,γ-di-[3-(iodomethyl)-3-butanol-1-yl]-triphosphate (diIHTP)
3,4,-epoxy-3-methyl-1-butyl-diphosphate (Epox-PP)
3,4,-epoxy-3-methyl-1-butyl-triphosphate (Epox-PPP)
α,γ-di-3,4,-epoxy-3-methyl-1-butyl-triphosphate (di-Epox-TP)

In a further variant, aminobiphosphonates are being used, such as 1-hydroxy-3-(methylpentylamino)propylidene-biphosphonic acid.

In an other variant, the synthetic activator is (E)-4-hydroxy-3-methyl-but-2-enyl pyrophosphate, as disclosed in Hintz et al.

Alternatively, although potentially less efficient, other activators for use in the present invention are phosphoantigens disclosed in WO95/20673, isopentenyl pyrophosphate (IPP) (US5,639,653), as well as alkylamines (such as ethylamine, iso-propyulamine, n-propylamine, n-butylamine and iso-butylamine, for instance).

The dose of activator may be adapted by the skilled artisan, depending on the nature of the activator, its specific-activity, EC50, stability and/or pharmaco-kinetics, as well as on the type of subject, etc. Preferably, the activator is administered in a human subject at a dose comprised between 0.05 and 200 mg/kg, preferably between 0.5 and 80 mg/kg, more preferably between 1 and 60, even more preferably between 2 and 60 mg/kg. The activator may be administered as a single dose, at the beginning of the treatment, or distributed over several days. Unexpectedly, however, the invention shows that a significantly higher effect is obtained when the activator is administered in a single dose at the beginning of the treatment. Typical dosages are comprised between 5 and 60 mg/kg, more preferably between 10 and 50 mg/kg. Appropriate dosages may be deduced from experiments conducted in animals, by normalizing with respect to the mean body weight and mean body surface. For instance, it can be calculated that efficient doses of between 2 and 300 mg/kg in a cynomolgus monkey (having a mean body weight of about 3kg and mean body surface of about 0.3 m²) correspond to an efficient dosage of between 0.5 and 80 mg/kg in a human subject (having a mean body weight of about 65kg and mean body surface of about 1.8 m²). It should be understood that different dosages may be used, including higher dosages, considering the low toxicity observed in vivo with the synthetic activators.

A specific embodiment of the present invention relates to the use of (i) a synthetic γδT activator selected from a PED or a PHD compound and (ii) an interleukin-2 polypeptide, for the manufacture of a pharmaceutical composition for treating cancer, an infectious disease or an auto-immune disease in a subject, wherein said synthetic γδT activator and interleukin-2 polypeptide are administered separately to the subject. More preferably, the interleukin-2 polypeptide is administered at low doses, typically over a period of time comprised between 1 and 10 days, and/or the synthetic γδT activator is administered in a single dose at the beginning of the treatment at a dose comprised between 2 and 60.

### The cytokine

As indicated above, the method is based on the use of particular combinations of active agents, according to particular schedules. The invention more preferably uses a cytokine in combination with a synthetic activator, the cytokine being an interleukin-2 polypeptide.

The interleukin-2 polypeptide may be of human of animal origin, preferably of human origin. It may comprise the sequence of a wild-type human (or animal) IL-2 protein, or any biologically active fragment, variant or analogue thereof, i.e., any fragment, variant or analogue capable of binding to an IL-2 receptor and of inducing activation of γδT cells in the method of this invention.

The sequence of reference, wild-type human interleukin-2 proteins is available in the art, such as in Genbank, under accession numbers NP000577 ; AAK26665 ; PO1585 ; XP035511, for instance.

The term « variant » designates, in particular, any natural variants, such as those resulting from polymorphism(s), splicing(s), mutation(s), etc. Such naturally-occurring variants may thus comprise one or several mutation, deletion, substitution and/or addition of one or more amino acid residues, as compared to a reference IL-2 protein sequence.

The term «variant» also includes IL-2 polypeptides originating from various mammalian species, such as for instance rodent, bovine, porcine, equine, etc. More preferably, the IL-2 polypeptide is of human origin, i.e., comprises the sequence of a human IL-2 protein or a variant, fragment or analogue thereof.

The term « variant » also includes synthetic IL-2 variants, such as any synthetic polypeptide comprising one or several mutation, deletion, substitution and/or addition of one or more amino acid residues, as compared to a reference IL-2 protein sequence, and capable of binding to an IL-2 receptor and of inducing activation of γδT cells in the method of this invention. Preferred synthetic IL-2 variants have at least 75% identity in amino acid sequence with the primary sequence of an IL-2 reference protein, more preferably at least 80%, even more preferably at least 85 or 90%. The identity between sequences may be determined according to various known methods such as, typically, using the CLUSTAL method.

Variants also include IL-2 polypeptides encoded by a nucleic acid sequence that hybridize, under conventional, moderate stringency, with the nucleic acid sequence encoding a reference IL-2 protein, or a fragment thereof. Hybridization conditions are, for instance incubation at 40-42°C for 12 hours in 50% formamide, 5 X SSPE, 5 X Denhardt's solution, 0.1 % SDS.

The IL-2 polypeptide may also be any fragment of a reference IL-2 protein which retain the ability to bind to an IL-2 receptor and to induce activation of γδT cells in the method of this invention. Such fragments contain, at least, one functional domain of IL-2, such as the receptor binding site. Fragments contain preferably at least 40%, 50% or, preferably, at least 60% of the complete reference sequence.

Analogues designate polypeptides using the same receptor as Interleukin-2 and thus mediating similar activation signal in a γδ T lymphocyte.

The interleukin-2 polypeptide may further comprise heterologous residues added to the natural sequence, such as additional amino acids, sugar, lipids, etc. This may also be chemical, enzymatic or marker (e.g., radioactive) groups. The added residues or moiety may represent a stabilizing agent, a transfection-facilitating agent, etc.

The IL-2 polypeptides may be in soluble, purified form, or conjugated or complexed with an other molecule, such as a biologically active peptide, protein, lipid, etc. The IL-2 polypeptide may be produced according to techniques known in the art, such as by chemical synthesis, enzymatic synthesis, genetic (e.g., recombinant DNA) synthesis, or a combination thereof. An IL-2 polypeptide of pharmaceutical grade may also be obtained from commercial sources.

The interleukin-2 polypeptide is preferably administered at low doses, i.e. at doses that are sufficient to target in vivo cells that express the high affinity receptor for IL2, defined as the tri-molecular complex CD25/CD122/CD130. Practically, in human, such doses have been experimentally defined in clinical trials as being comprised between 0.2 and 2 million units per square meters, when injected subcutaneously (see for example buzio et al 2001).

The IL-2 polypeptide is preferably administered by injection of between 0.1 and 3 Million Units per day, over a period of 1 to 10 days. Preferably, daily doses of between 0.2 and 2 MU per day, even more preferably between 0.2 and 1.5 MU, further preferably between 0.2 and 1 MU, are being administered. The daily dose may be administered as a single injection or in several times, typically in two equal injections. The IL-2 treatment is preferably maintained over between 1 and 9 days, even more preferably during 3 to 7 days. Optimum effect seems to be achieved after 5 days treatment.

### Mode of use

The present invention stems from the definition of particular combinations and particular conditions that allow efficient expansion and biological activation of γδ T cells in vivo, in a subject. As disclosed above, the invention more preferably uses a synthetic γδ T cell activator and an interleukin-2 polypeptide, which are separately administered.

Within the context of the present invention, the term "separately administered" indicates that the active ingredients are administered at a different site or through a different route or through a different schedule to the subject. Accordingly, the ingredients are generally not mixed together prior to administration, although they may be combined in a unique package in suitable separated containers.

In a preferred embodiment, the active ingredients are administered through different schedules: the synthetic γδ T cell activator is administered as a single shot, at the beginning of the treatment, and the interleukin-2 polypeptide is administered over a prolonged period of time, typically between 1 and 10 days. As shown in the experimental section, such administration schedule provides a remarkable increase in the activity of γδ T cells in a subject.

The active ingredients may be administered through different routes, typically by injection or oral administration. Injection may be carried out into various tissues, such as by intravenous, intra-peritoneal, intra-arterial, intra-muscular, intra-dermic, subcutaneous, etc. Preferred administration routes for the synthetic activators are intravenous and intra-muscular. Preferred administration routes for the cytokine are subcutaneous, intravenous and intra-muscular.

The above treatment may be repeated several times, to re-stimulate the activity of γδ T cells in a subject. As shown in the experimental section, repeated treatments may be performed at various intervals, each leading to a remarkable increase in the activity of γδ T cells, and without toxicity to the subject.

Further aspects and advantages of the present invention will be disclosed in the following experimental section, which should be regarded as illustrative and not limiting the scope of the present application.

### 1. Legend to the Figures

### Figure 1

Successive intravenous injections of increasing doses of BrHPP do not sustain γδ cell amplification in the periphery of M. fascicularis. Two animals received successively 1, 4, 16 and 32mg/kg BrHPP daily and their PBMCs were monitored by flow cytometry. Total blood was stained with anti-delta2-FITC and anti-CD3-PE antibodies until 20 days after BrHPP administration.

### Figure 2

**A- BrHPP and IL2 co-administration induces reproducible** γδ **cell increase in *M*. *fascicularis*.** Two animals received 20mg/kg BrHPP ("1 dose") and two others received 4mg/kg BrHPP daily during 5 days ("split doses"). All of them were co-injected with 0.9 million UI IL2 per day for 5 days. The four BrHPP/IL2 co-treated animals showed an increase in peripheral γδ cells, with a peak at day 7. As a control, a fifth animal was treated with IL2 alone and exhibited no change in its peripheral γδ rate. A BrHPP/IL2 co-administration to this animal at day 14 demonstrated it was able to answer with the same increase in γδ rate 7 days after. **B- Flow cytometry analyses of total blood of *M*. *fascicularis* at day 7 after administration.** These graphs represent the anti-delta2-FITC/anti-CD3-PE staining on total blood of the animals treated with IL2 alone (Z604 D7 IL2 only) or with BrHPP and IL2 (Z604, X973, Z059, Z135, Z714, D7 IL2+BrHPP) 7 days after the administration. **C- Absolute number of circulating γδ cells increases 5- to 40-fold in BrHPP/IL2 co-injected *M. fascicularis*.** Absolute numbers of peripheral γδ cells were determined by flow cytometry in the four BrHPP/IL2 co-injected animals. Peripheral γδ cell number was found 5 to 40 times higher at day 7 after administration than before, depending on animals. A mean 24-fold increase was obtained for a total amount of 20mg/kg BrHPP injected, single doses being more efficient than split ones.

### Figure 3

**A Peripheral γδ cell rate increase upon BrHPP/IL2 administration is dose-dependant.**

**Upper panel :** A first injection was done with increasing amounts (0, 0.2, 4, 20, 80 mg/kg) of BrHPP in 10 animals (numbered 2031 to 2040), with 2 animals per dose (1 male + 1 female).

**Lower panel :** Same animals underwent a second injection 3 weeks after the first injection with even higher amounts of BrHPP (20, 80, 120, 160 mg/kg). Animals that received the lowest doses at first injection (groups 0.2 and 4 mg/kg) were injected with the two new highest doses (120 and 160 mg/kg) to minimize potential effects of first injection on second injection.

**B- Circulating** γδ **cell number increase upon BrHPP/IL2 co-treatment is also dose-dependant.** The fold increase in peripheral γδ cell number was calculated as the ratio of γδ absolute count 7 days after treatment on γδ absolute count before treatment. Despite some discrepancy between animals in the same dose-group, this increase is clearly dose-dependant.

**C- Flow cytometry images of total blood of animal #2034, before and 5 days after it received 160mg/kg BrHPP,** stained with anti-gamma9-FITC and anti-CD3-PE antibodies.

### Figure 4

**A- Primate γδ cells respond *in vivo* equally to the first and the second BrHPP/IL2 co-treatment.** The mean amplification in γδ cell number observed 7 days after the first 20mg/kg BrHPP treatment (fractionated or not) is comparable to the mean increase observed after the 20mg/kg BrHPP recall.

**B- y8 amplification *in vivo* decreases after successive BrHPP/IL2 administration.** In this experiment, two animals received successively 80mg/kg and 22 days after 20mg/kg, or 20mg/kg followed by 80mg/kg. In each case, the response in γδ cell number fold increase is lower at the first recall of a given dose than at first injection (left panel). Moreover, the five females were treated with a second and a third recall at 80mg/kg, at 3 weeks intervals. The resulting amplification rates is still detectable but become lower at each recall (right panel).

### Figure 5

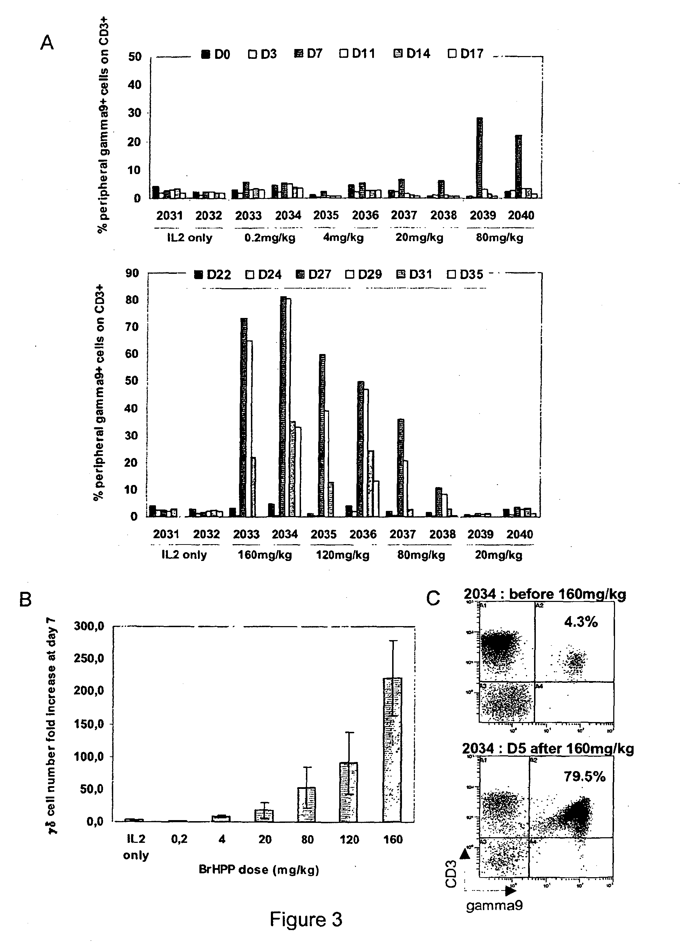

**A- Serum TNFα is produced in *vivo* after BrHPP/IL2 co-treatment.** TNFα was detected by Elisa in the serum of the monkeys first treated with IL2 and increasing doses of BrHPP (0 to 80mg/kg, with two animals per dose, Cf figure 3A), 1 and 4 hours after BrHPP injection.

**B- *In vivo* peripheral BrHPP-amplified primate** γδ **cells produce cytokines in response to BrHPP challenge.** As a fourth BrHPP/IL2 treatment, females 2032 and 2034 received 80mg/kg BrHPP, which induced 3- and 7.9-fold increases in γδ cell number respectively. At the time of γδ cell peripheral peak (day 5 after the 4^{th} injection), these animals were re-injected with 80mg/kg BrHPP (without IL2) and serum INFγ and TNFα were detected 60 and 120 minutes after injection by Elisa.

### Figure 6

**Different IL2 co-treatment do not influence the maintenance of peripheral gd rate.** Concomitantly with 20mg/kg BrHPP, animals received subcutaneously the following IL2 treatment: 0.15 million units twice daily for 9 days (animal Z059), 0.3 million units twice daily for 5 days (Z135), 0.9 million units twice daily for 5 days (animal Z714) or 9 days (animal X973).

### 2. Material and methods

### 2.1. Animals

- Group 1 : 5 purpose bred healthy male cynomolgus monkeys (M. fascicularis), supplied by C.R.P. Le Vallon, Ferney S.E., Mahebourg, Mauritius. At the beginning of the study, body weights range from 3.7 to 4.6 kg.
- Group 2 : 10 purpose bred healthy cynomolgus monkeys (5 males and 5 females), supplied by C.R.P. Le Vallon. At the beginning of the study, body weights range from 1.8 to 3.5 kg and ages from 2 to 3 years.
   Husbandry conditions conformed to the European requirements, comprising monitored temperature, humidity, air change and lighting cycle. Group 1 animals were housed in Biomatech (Chasse sur Rhône, France), and group 2 animals were housed in MDS, (Les Oncins, France).
   All experiments were subjected to local ethical committee before processing.

### 2.2. Phosphoantigens

The synthesis and characterization of trisodium (R,S)-3-(bromomethyl)-3-butanol-1-yl-diphosphate BrHPP has been described previously (Espinosa, 2001). The lot used for the experiments described here was manufactured and characterized under GMP conditions by PCAS-SELOC (Limay, France). Sterilization and clinical unit preparation was conducted under GLP by AXCELL BIOTECHNOLOGIES (Saint-Genis l'Argentière, France). Titration of BrHPP in sterile aqueous solution was achieved by High Performance Anion-Exchange Chromatography with conductimetric detection (DIONEX DX600 system).

### 2.3. Drug administration and blood sampling

- Group 1 : animals were anaesthetised with intra-muscular injection of 6mg/kg ZoletilND 100 (Tiletamine-Zolazepam, Virbac, Carros, France) before any injection or blood taking.
- Group 2 : injections and blood taking were performed on manually restrained non-anaesthetised animals.
- 4-day BrHPP alone treatment (group 1) : 2 animals received 1mg/kg of BrHPP in 10ml saline on day 0 (microflex infusion set introduced into the external saphenous vein) and then 4mg/kg, 16mg/kg and 32mg/kg in 20ml saline on days 1, 2 and 3 by the same way. (Duration of infusion : 10 to 15 min).
- BrHPP/IL2 co-treatments (group 1) : 5 animals received either 20mg/kg once or 4mg/kg 5 times daily of BrHPP in final 50ml saline by the same way as above (duration of infusion : 30 min). IL2 (18 million UI per vial, Proleukin®, Chiron, US) was resuspended in 1ml sterile water and diluted to 10ml with 4% HSA for a final concentration of 1.8 million UI/ml. In a first cycle of treatment, all animals received the same dose of IL2 consisting of 5 days of twice daily injections of 0.9 million units IL2.
   In a second cycle of treatment, animals received subcutaneously the following IL2 treatment : 0.15 million units twice daily for 9 days (animal Z059), 0.3 million units twice daily for 5 days (Z135), 0.9 million units twice daily for 5 days (animal Z714) or 9 days (animal X973).
   A single animal received 80 mg/kg BrHPP + 9 days of IL2 co-treatment consisting of a single daily subcutaneous injection of 0.6 million units.
- BrHPP/IL2 co-treatments (group 2) : BrHPP was diluted in saline to the appropriate final concentration (depending on the dose to inject and on the last recorded body weight) so as to inject always approximately 50ml in 30min (microflex infusion set introduced into cephalic or external saphenous vein). All animals received 0.6 million UI IL2 per day for 7 days. IL2 was administered subcutaneously as 2 separate injections of 0.3 million UI IL2 in sterile water, 8-hour apart. Control animals received IL2 only.
   Twice weekly blood samples (1 to 4ml) were withdrawn from femoral vessels/artery into EDTA containing tubes. Tubes were shipped overnight at room temperature (RT) before flow cytometry analyses.

### 2.4. Flow cytometry

Peripheral γδ lymphocytes were analysed twice weekly by flow cytometry on total monkey blood, after double staining with anti-CD3-PE antibody and anti-Vgamma9-FITC antibodies and/or anti Vd2 antibodies (CD3-PE : SP34 clone, BD Biosciences Pharmingen, Le Pont de Claix, France). Anti Vgamma 9, clone 7B6 is a monoclonal raised to human Vgamma 9 but that cross-reacts with cynomolgus cells. It was purified by affinity chromatography on protein A and coupled to FITC as previously described. We checked that this antibody stained most of Vd2 positive cells, stained by commercial (Endogen, Woburn, MA) TCR2732 clone (as previously described for other Vg9 antibodies in Rhesus monkey by Shen, 2002, data not shown). Of note, the anti Vd2 was stopped by manufacturer during the course of this study.
Briefly, 50µl monkey blood is incubated 15 min at RT with 5µl anti-CD3-PE and 6µl anti-delta2-FITC or 10µl anti-gamma9-FITC antibodies. Antibodies are washed with 3ml 1X PBS, centrifuged for 4 min at 1300rpm at RT and supernatant is discarded. Red cells are lysed with the OptiLyse C reagent (Immunotech-Beckman-Coulter, Marseilles, France) according to the manufacturer's instructions. At the final step, stained white blood cells are recovered by centrifugation and resuspended in 300µl PBS + 0.2% PFA. Immediately before analysis, 50µl calibrated Flow CountTM Fluorospheres (Immunotech-Beckman-Coulter, Marseilles, France) are added to the cells for absolute number counting of the populations of interest.
For group 2, lymphocyte subsets were also analysed in parallel by dual colour flow cytometry with CD20-FITC (2H7 clone); CD3-PE (SP34 clone); CD4-FITC (M-T477 clone); CD8-FITC (SK1 clone) (all purchased from BD Biosciences, Le Pont de Claix, France).
Flow cytometry was performed on a Epics XL-MCL apparatus (Beckman-Coulter, Roissy, France) with the Expo32 software.

### 2.5. Cytokine detection

Serum cytokines (TNFa and INFg) were detected and quantified with the BIOSOURCE CytoscreenTM ELISA monkey TNFa and CytoscreenTM ELISA monkey INFg respectively (purchased from CliniSciences, Montrouge, France) according to the manufacturer's instructions.

### 2.6. Haematology and serum clinical chemistry

Classical blood parameters follow-up (red blood cell, platelets total and differential white blood cells counts, haemoglobin, mean corpuscular haemoglobin, mean corpuscular haemoglobin concentration) were performed at the sites of monkey handling, just before and after each administration and twice weekly.
On all animals in group 2, 48 or 72 hours after each injection, 16 blood chemistry parameters were measured (sodium, potassium, chloride, calcium, inorganic phosphorus, glucose, urea, total cholesterol, total bilirubin, total protein, albumin, globulin, creatinin, alkaline phosphatase, aspartate aminotransferase and alkaline aminotransferase).

### 2.7. Vital parameters follow up

The animals were observed daily and during and after each injection for any change in vital and clinical parameters (general behaviour, skin, hair, respiratory system, central nervous system). Animals were regularly weighed, every 3 days (group 1) or weekly (group 2). Body temperature (on vigil animals of group 2) was measured before and at the end of each BrHPP/IL2 (or IL2 alone) infusion and once daily during the 5 days following administration. Heart rate and blood pressure were recorded for all animals in group 2, before and at the end of each administration.
All animals were observed at least twice daily for signs of morbidity/mortality.

### 2.8. Biopsy preparations

5 animals in group 2 were sacrificed 9 days after the second administration, by intravenous injection of sodium pentobarbitone, exsanguinated, and were submitted to full necropsy procedures for organ toxicology assessment of the drug. Samples of organs were weighed and collected in RPMI medium (Gibco-BRL - Life Science) for further processing.
Lymphoid organ samples (thymus, tonsils, bone marrow, mesenteric, inguinal and tracheo-bronchial lymph nodes) were carefully mechanically dissociated with sterile syringe plungers, washed several times in RPMI medium and filtered twice through nylon membranes (Scrynel NYHC 100µm nylon, purchased from VWR International, France). Cells were then double stained with anti-CD3-PE and anti-gamma9-FITC antibodies and analysed by flow cytometry as described above.

### 3. Results.

### 3.1. BrHPP alone does not induce reproducible expansion of g9d2 cells in vivo

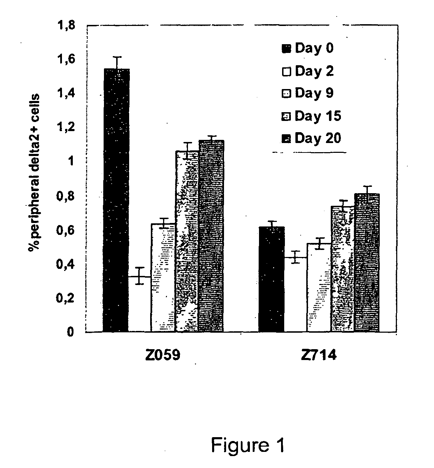

We tested first if BrHPP injection alone was able to support activation of g9d2 positive T cells in vivo.
In a first series of experiment, 4 monkeys were treated by five daily injection of 0.2 mg/kg of BrHPP, and 4 animals were treated with saline. Injection of this dose of BrHPP did not result in any toxicity in the treated non human primates as assessed by vital signs or rectal temperature. Follow up of Vg9Vd2 T cells showed a slight increase in % of Vg9Vd2 in two treated animals and none in the placebo group but this expansion was not significant (data not shown).
To test if higher doses of the molecule could induce a more consistent activation or expansion, another two animals received four daily injections with increasing dosing of BrHPP (1mg/kg, 4 mg/kg, 16 mg/kg and 32 mg/kg).
Again no toxicity nor fever was observed in the two treated animals. Follow up of Vg9Vd2 reavealed a decrease at day two post first injection, but the two animals returned to basal level at day 9 (figure 1). Survey of CD25 and CD69 did not give significant results, but these markers may not be optimal for study of non human primate cells.
At least two possibities can explain the lack of objective response of Vg9Vd2 cells upon BrHPP treatment i) as the BrHPP is a small molecule containing pyrophosphate, it may be excreted or degraded so rapidly that it does not allow sufficient contact with the target cells to allow activation of the cells ii) Expansion of g9d2 cells in vivo requires, as in vitro, the presence of cytokine, particularly IL2.
Although we do not have at present an analytical method sensitive enough to follow the molecule in vivo, it was likely that the serum concentration in vivo would be sufficient to trigger Vg9Vd2 cells. In vitro, we and others (Lang, 1995) have shown that the activation of Vg9Vd2 cells occurs within minutes after the addition of the molecule. Two mechanisms may prevent the molecule to be active in vivo i) the likely rapid renal clearance of such a small molecule and ii) a degradation process in vivo. The study of the half life of the molecule in primate sera showed that the half life of the molecule at 37°C is about one hour. The main mechanism of degradation of the molecule is the removal of the first phosphate, rendering the molecule biologically inactive. Nevertheless, the concentration of the molecule, injected by intravenous route in relatively high amount, should be maintained beyond the 20nM EC50 for a significant time, if no additional, blood cell or organ related degradation process occurs in vivo.
To try to reveal the activation of the cells, we tested the concomitant injection of low doses of rhIL2 with BrHPP.

### 3.2. BrHPP + IL2 induces reproducible, transient expansion of Vg9Vd2 cells in vivo in cynomolgus.

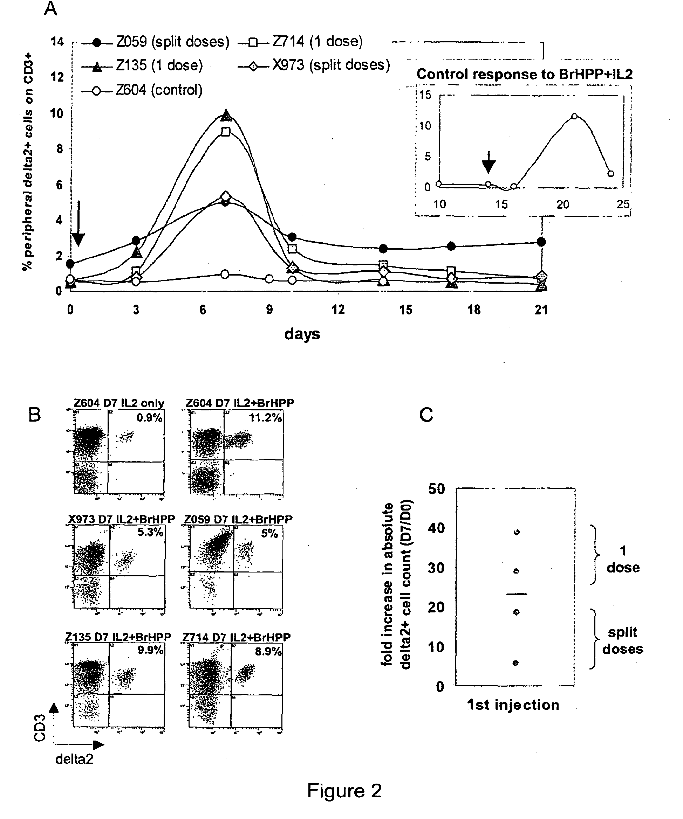

4 animals and one control (group 1 animals) were treated with the combination of BrHPP or saline with low doses of rhIL2. This group of animals was composed of the two previous animals that did not show any augmentation of Vg9Vd2 upon injection of BrHPP alone and three new animals. Among the four BrHPP treated animals 2 animals underwent 5 daily injection of 4mg/kg of BrHPP, and two animals underwent a single injection of 20 mg/kg. The BrHPP treated animals and control were administered the same rhIL2 regimen consisting of 0.9 million units twice daily by subcutaneous injection for 5 days. The control animal was treated first with saline and IL2, and then 14 days later with a single shot of BrHPP and a second course of 5 days IL2.
Haematological follow up of the animals revealed increase of lymphocyte counts by 2 to 4 in the BrHPP treated animals and control as well, consistent with the IL2 administration.
A specific expansion both in percent increase among CD3 positive cells and absolute numbers of Vg9Vd2 was observed in all BrHPP treated animals (fig2). This increase was transient, as the expansion is seen at day 7 with already a slight increase at day 3, and levels being back to around before treatment level at day 10. The control animal treated with IL2 alone, showed a slight increase in absolute numbers but no increase in percentage, suggesting that peripheral Vg9Vd2, as other lymphocyte subset, slightly increase upon IL2 administration. Administration of BrHPP on the control animal, after this cycle of IL2 alone, results in an expansion consistent with that of treated animals, therefore validating this animal as a control.
Absolute increase of Vg9Vd2 was higher in the two animals receiving a single dose of BRHPP (x30 and x40) as compared to animals receiving the same dose but split in 5 daily injections (x8 and x18) (figure 1c). This may be due either to the deleterious effect of multiple injection or lower individual dose injected. As single injection was at least as efficient and more practical, next experiments were performed with single injection of the product.

### 3.3. Dose range effect of BrHPP in cynomolgus monkey

To evaluate the dose range effect of the molecule, a new experiment was set on 10 new animals (group 2 animals). Subroups of two animals, one male and one female, were treated with increasing doses of BrHPP (0, 0.2, 4, 20, 80 mg/kg), and co-treated with IL2 for 7 days.
Again, animals treated with IL2 alone did experience a slight increase of Vg9Vd2 cells, of the same order of magnitude as compared to other lymphocyte subset. 0.2 mg/kg dose was undistinguishable from the control animals, both in terms of % and absolute numbers of Vg9Vd2. A dose range effect was observed both in % and absolute numbers of Vg9Vd2 from 4 to 80 mg/kg (figure 3a) at day 7, without apparently reaching a plateau. Again % and absolute counts came rapidly back to pre-treatment level.
As a plateau was not reached during this treatment, same animals were treated a second time 21 days post first injection, after Vg9Vd2 came back to pre-treatment level, with doses ranging from 20 mg/kg, 80 mg/kg, 120 mg/kg and 160 mg /kg. To minimize potential effect of the first treatment on the second treatment, the new doses (120 and 160 mg/kg) were given to animals that seldom responded to the first treatment (ie animals treated with 0.2, and 4mg/kg during the first course of treatment, figure 3b).
The time line of the proliferation after this second injection was about the same as described before. It should be noted that at the highest concentration, the level of circulating Vg9Vd2 reached about 80 % of circulating CD3 positive cells (figure 3b), with absolute numbers reaching a mean of 10,000 Vg9Vd2 cells/mm3. The numbers and percentage of Vg9Vd2 declined after the peak between day 5 and day 7 , although the time to go back to pre-injection level appears slower for the two highest doses.
Pooled data from injection 1 and injection 2 gave a clear dose range effect both in terms of percentage and absolute numbers of Vg9Vd2 cells at the peak of response (figure 3c and 3d). This dose range effect is observed despite some individual heterogeneity, particularly in the 120mg/kg group, as demonstrated by the error bars, with a no effect dose situated between 0.2 and 4 mg/kg, still higher doses would have been necessary to establish a plateau.

### 3.4. Influence of IL2 schedule

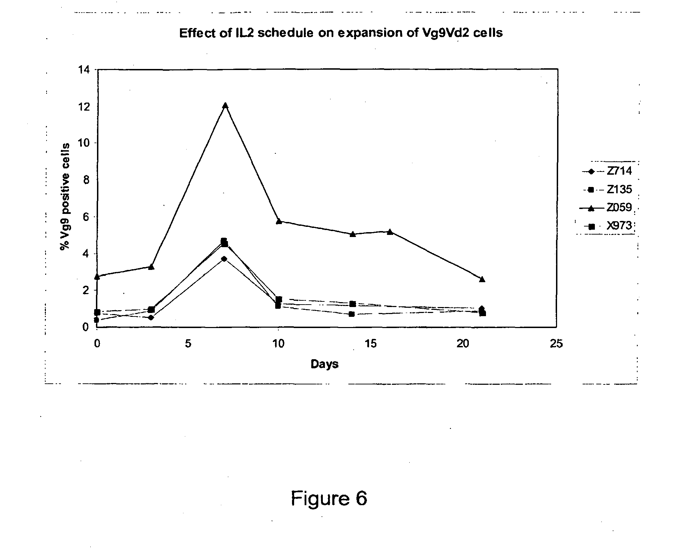

5 animals from group 1 were submitted to injections of BrHPP followed by different doses and time schedule of IL2. As shown in figure 6, even doses as low as 0.15 million units twice daily are sufficient to support expansion of Vγ9Vδ2 T cells, apparently equivalent to higher doses (0.3 and 0.9 million units twice daily). The length of the Vγ9Vδ2 expansion does not seem to increase when IL2 treatment is given for more than five days, although more animals should be tested to clearly establish it. Interestingly, an animal subjected to 0.6 million units subcutaneous injection of IL2 as a single daily injection also developed an expansion of Vγ9Vδ2 cells (data not shown).

### 3.5. Phosphoantigen + IL2 induced expansion can be reproduced at least five times successively

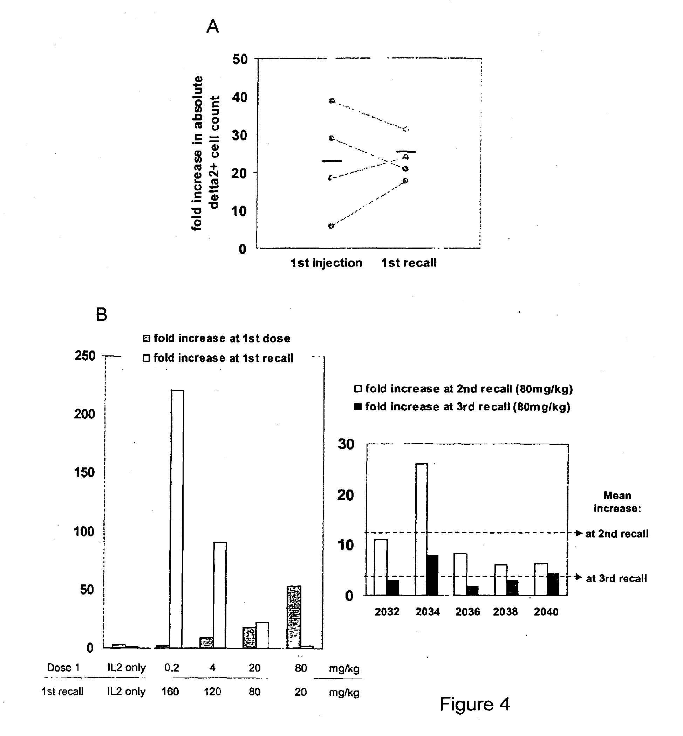

A memory type response to a recall injection of phosphoantigen containing preparation (ie BCG) was recently suggested in a primate model. We tested if such a response could be obtained with a pure preparation of synthetic ligand, and IL2 cotreatment.
Animals from group 1 were treated a second time with a single shot of 20 mg/kg and 7 days IL2 and Vg9Vd2 increase was monitored by flow cytometry. Fold increase obtained with the two injections is represented in figure 4a. The timing of the amplification of Vg9Vd2 was the same as in the first injection. The two animals that were treated with a single shot of BrHPP during the first injection showed a slightly reduced amplification rate in the second injection. The two animals that were treated with 5 daily 4mg/kg of BrHPP injections showed a slightly higher amplification rate at the second injection, most probably reflecting the better effectiveness of single injections over fractionated injections as discussed before.
As mentioned before, animals of group 2 underwent two successive injections during the dose range assay. Interestingly, the two animals that were treated first with 80 mg/kg, and then with 20 mg/kg showed a decreased expansion, as compared to all animals tested with a first injection of BrHPP at 20 mg/kg (fig 4b). Animals treated first with 20 mg and then with 80 mg showed expansions comparable to animals treated with 80 mg/kg at the first injection. Taken together, these results suggest that response of Vg9Vd2 to BrHPP + IL2 expansion can be repeated twice, although may be with lower expansion rate.
To get further insight into the possible influence of successive injections on the Vg9Vd2 response, 5 animals from group 2 (one animal per subgroup) were treated with a third cycle of BrHPP injection 21 days after the second injection and a fourth cycle after another 21 days.
Again the kinetic of Vγ9Vδ2 expansion was the same as described previously, with a peak between day 5 and day 9. As shown in figure 3c, one animal gave a high increase at the third injection (x 28). Interestingly, this animal was the only one which did not come back to the basal level after the second injection of 120mg/kg (animal 2034, fig3B). The control animal which received only IL2 in the first two courses of treatment gave an increase of around 40, compatible with the values obtained in four animals treated with this dose in the first two treatment cycles (fig 4b). The three other animals gave amplification lower than 40, lower than amplification in animals treated with the same dose at injection 1 or 2.
The five animals treated with a fourth injection of 80 mg/kg BrHPP and IL2 gave a detectable increase of Vg9Vd2 although lower than that obtained in the third injection (fig 4b).
In conclusion, it is shown that induction of proliferation of Vg9Vd2 by BrHPP + IL2 co-treatment can be reproduced several times. In these experiments, we could achieve expansion 4 successive times, with injections of BrHPP separated by 20 days or higher.

### 3.6. Short term production of cytokine by g9d2 T cells in vivo upon challenge with Phosphoantigens

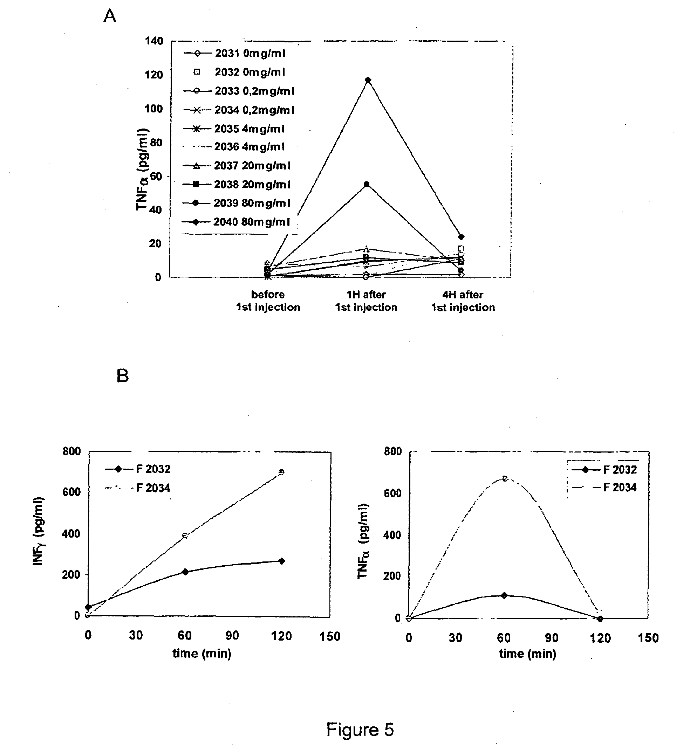

Vg9Vd2 cells are known producers of TNFa and IFNg in vitro upon phosphoantigen challenge. In order to evaluate if these cells could be a source of these cytokines in vivo, sera of some animals was collected shortly after BrHPP injection.
Samples of sera were collected from group 2 animals during the first dose range assay (0 to 80 mg/kg) just before injection then 1 and 4 hours post injection, and assayed by ELISA specific for TNFa and IFNg.
Assessment of IFNγ did not give significant results in all treated animals.
TNFα was detectable in the sera of animals treated at the highest dose (80 mg/kg) one hour after injection of BrHPP (fig. 5a). The serum level of TNFa rapidly decreased as it was not further detectable 4 hours post BrHPP injection and remains undetectable during the increase of Vg9Vd2 in blood. This suggests that TNFa is rapidly produced from intracellular, pre-formed pool by Vg9Vd2, and then is seldom produced after the initial activation by the drug.
To test the capacity of cells to produce cytokines after expansion with the drug, two animals of group 2 were injected a fifth time at 80 mg/kg with BrHPP (without IL2) at day 7 post fourth 80 mg/kg injection, where the level of Vg9Vd2 was at the peak. As shown in figure 5B, both TNFa and IFNg was detectable in the sera of treated animals. Production of TNFa followed the same kinetic as compared to the first experiment, with a level becoming undetectable at 4 hours post injection. On the reverse, IFNg became detectable at one hour post injection, and increased at 4 hours post injection, suggesting a slower, but more sustained production of this cytokine.

### 3.7. Absence of toxicity of injection of BrHPP alone or in association with IL2

No alteration of clinical or blood chemical parameters (see material and methods) were seen in any of the animals treated either with BrHPP alone or in association with IL2. From an haematologic point of view, in all animals treated with IL2, a transient increase (2 to 5 times) in lymphocytes was observed. In animals treated with the highest dose of BrHPP, a lymphocytosis was observed, corresponding to the peak of Vg9Vd2 in the periphery.

It should be noted that rectal temperature was not significantly affected in any animal treated, despite the presence of detectable level of TNFa and IFNg inflammatory cytokines in sera of some treated animals.

### REFERENCES

Buzio, C., Andrulli, S., Santi, R., Pavone, L., Passalacqua, R., Potenzoni, D., Ferrozzi, F., Giacosa, R., and Vaglio, A. (2001). Long-term immunotherapy with low-dose interleukin-2 and interferon-alpha in the treatment of patients with advanced renal cell carcinoma. Cancer 92, 2286-2296.

Constant, P., Poquet, Y., Peyrat, M. A., Davodeau, F., Bonneville, M., and Fournie, J. J. (1995). The antituberculous Mycobacterium bovis BCG vaccine is an attenuated mycobacterial producer of phosphorylated nonpeptidic antigens for human gamma delta T cells. Infect Immun 63, 4628-4633.

Espinosa, E., Belmant, C., Pont, F., Luciani, B., Poupot, R., Romagne, F., Brailly, H., Bonneville, M., and Fournie, J. J. (2001 a). Chemical synthesis and biological activity of bromohydrin pyrophosphate, a potent stimulator of human gamma delta T cells. J Biol Chem 276, 18337-18344.

Espinosa, E., Belmant, C., Sicard, H., Poupot, R., Bonneville, M., and Fournie, J. J. (2001b). Y2K+1 state-of-the-art on non-peptide phosphoantigens, a novel category of immunostimulatory molecules. Microbes Infect 3, 645-654.

Fisch, P., Moris, A., Rammensee, H. G., and Handgretinger, R. (2000). Inhibitory MHC class I receptors on gammadelta T cells in tumour immunity and autoimmunity. Immunol Today 21, 187-191.

Fournie, J. J., and Bonneville, M. (1996). Stimulation of gamma delta T cells by phosphoantigens. Res Immunol 147, 338-347.

Hayday, A. C. (2000). [gamma] [delta] cells: a right time and a right place for a conserved third way of protection. Annu Rev Immunol 18, 975-1026.

Hintz et al., (2001). FEBS Lett. Dec 7;509(2):317-22.

Kunzmann, V., Bauer, E., Feurle, J., Weissinger, F., Tony, H. P., and Wilhelm, M. (2000). Stimulation of gammadelta T cells by aminobisphosphonates and induction of antiplasma cell activity in multiple myeloma. Blood 96, 384-392.

Kunzmann, V., Bauer, E., and Wilhelm, M. (1999). Gamma/delta T-cell stimulation by pamidronate. N Engl J Med 340, 737-738.

Lang, F., Peyrat, M. A., Constant, P., Davodeau, F., David-Ameline, J., Poquet, Y., Vie, H., Fournie, J. J., and Bonneville, M. (1995). Early activation of human V gamma 9V delta 2 T cell broad cytotoxicity and TNF production by nonpeptidic mycobacterial ligands. J Immunol 154, 5986-5994.

Miyagawa, F., Tanaka, Y., Yamashita, S., and Minato, N. (2001). Essential requirement of antigen presentation by monocyte lineage cells for the activation of primary human gamma delta T cells by aminobisphosphonate antigen. J Immunol 166, 5508-5514.

Morita, C. T., Beckman, E. M., Bukowski, J. F., Tanaka, Y., Band, H., Bloom, B. R., Golan, D. E., and Brenner, M. B. (1995). Direct presentation of nonpeptide prenyl pyrophosphate antigens to human gamma delta T cells. Immunity 3, 495-507.

Poccia, F., Cipriani, B., Vendetti, S., Colizzi, V., Poquet, Y., Battistini, L., Lopez-Botet, M., Fournie, J. J., and Gougeon, M. L. (1997a). CD94/NKG2 inhibitory receptor complex modulates both anti-viral and anti-tumoral responses of polyclonal phosphoantigen-reactive V gamma 9V delta 2 T lymphocytes. J Immunol 159, 6009-6017.

Poccia, F., Malkovsky, M., Gougeon, M. L., Bonneville, M., Lopez-Botet, M., Fournie, J. J., and Colizzi, V. (1997b). Gammadelta T cell activation or anergy during infections: the role of nonpeptidic TCR ligands and HLA class I molecules. J Leukoc Biol 62, 287-291.

Rojas, R. E., Torres, M., Fournie, J. J., Harding, C. V., and Boom, W. H. (2002). Phosphoantigen presentation by macrophages to mycobacterium tuberculosis--reactive Vgamma9Vdelta2+ T cells: modulation by chloroquine. Infect Immun 70, 4019-4027. Shen, Y., Zhou, D., Qiu, L., Lai, X., Simon, M., Shen, L., Kou, Z., Wang, Q., Jiang, L., Estep, J., et al. (2002). Adaptive immune response of Vgamma2Vdelta2+T cells during mycobacterial infections. Science 295, 2255-2258.

## Claims

1. The use of a synthetic γδT activator and an interleukin-2 polypeptide, for the manufacture of a pharmaceutical composition for the treatment of a cancer, an infectious disease, an auto-immune disease or an allergic disease in a human subject, the synthetic γδT activator and interleukin-2 polypeptide being administered separately to the subject, said synthetic γδT activator being administered as a single dose at the beginning of the treatment at a dose comprised between 2 and 60 mg/kg and said interleukin-2 polypeptide being administered at low doses.

2. The use of claim 1, wherein the interleukin-2 polypeptide is administered over a period of time comprised between 1 and 10 days.

3. The use of claim 2, wherein the interleukin-2 polypeptide is administered at a daily dose comprised between 0.2 and 2 MU/m² per day, even more preferably between 0.2 and 1.5 MU/m², further preferably between 0.2 and 1 MU/m².

4. The use of any one of claims 1 to 3, wherein the daily dose of interleukin-2 polypeptide is administered as a single injection or in two injections.

5. The use of any one of the preceding claims, wherein the synthetic γδT activator is a ligand of the T receptor of γδT lymphocytes.

6. The use of claim 5, wherein the synthetic γδT activator is selected from phosphohalohydrin compounds, phosphoepoxyde compounds, biphosphonate compounds and alkylamines.

7. The use of claim 6, wherein the synthetic γδT activator is selected from the following compounds :
3-(bromomethyl)-3-butanol-1-yl-diphosphate (BrHPP)
3-(iodomethyl)-3-butanol-1-yl-diphosphate (IHPP)
3-(chloromethyl)-3-butanol-1-yl-diphosphate (CIHPP)
3-(bromomethyl)-3-butanol-1-yl-triphosphate (BrHPPP)
3-(iodomethyl)-3-butanol-1-yl-triphosphate (IHPPP)
α,γ-di-[3-(bromomethyl)-3-butanol-1-yl]-triphosphate (diBrHTP)
α,γ-di-[3-(iodomethyl)-3-butanol-1-yl]-triphosphate (diIHTP)
3,4,-epoxy-3-methyl-1-butyl-diphosphate (Epox-PP)
3,4,-epoxy-3-methyl-1-butyl-triphosphate (Epox-PPP)
α,γ-di-3,4,-epoxy-3-methyl-1-butyl-triphosphate (di-Epox-TP)

8. The use of any one of the preceding claims, wherein the synthetic γδT activator is a PED or PHD compound and is administered as a single injection at a dose comprised between 10 and 50 mg/kg, at the beginning of the treatment, and wherein the interleukin-2 polypeptide is administered at a daily dose comprised between 0.2 and 2 MU/m² per day over a period of time comprised between 1 and 10 days.

9. The use of any one of the preceding claims for treating cancer in a subject.

10. The use of any one of claims 1 to 8 for treating an infectious disease in a subject.

11. The use of anyone of claims 1 to 8 for treating an autoimmune disease in a subject.

12. The use of any one of claims 1 to 8 for treating a disease caused by or associated with pathological cells sensitive to lysis by γδT cells in a subject.

13. A product comprising a synthetic γδT activator and an interleukin-2 polypeptide, for separate use, for the treatment of a cancer, an infectious disease, an auto-immune disease or an allergic disease in a human subject, wherein said synthetic γδT activator is administered as a single dose at the beginning of the treatment at a dose comprised between 2 and 60 mg/kg and said interleukin-2 polypeptide is administered at low doses.

## Patentansprüche

1. Verwendung eines synthetischen γδT-Aktivators und eines Interleukin-2 Polypeptids zur Herstellung einer pharmazeutischen Zusammensetzung zur Behandlung von Krebs, einer infektiösen Erkrankung, einer Autoimmunerkrankung oder einer allergischen Erkrankung in einem humanen Subjekt, wobei der synthetische γδT-Aktivator und das Interleukin-2 Polypeptid dem Subjekt getrennt verabreicht werden, wobei der synthetische γδT-Aktivator als eine einzelne Dosis am Anfang der Behandlung in einer Dosis umfassend zwischen 2 und 60 mg/kg und das Interleukin-2 Polypeptid in niedriger Dosis verabreicht wird.

2. Die Verwendung nach Anspruch 1, wobei das Interleukin-2 Polypeptid über einen Zeitraum umfassend zwischen 1 und 10 Tagen verabreicht wird.

3. Die Verwendung nach Anspruch 2, wobei das Interleukin-2 Polypeptid in einer täglichen Dosis umfassend zwischen 0,2 und 2 ME/m² pro Tag verabreicht wird, sogar noch bevorzugter zwischen 0,2 und 1,5 ME/m², weiter bevorzugt zwischen 0,2 und 1 ME/m².

4. Die Verwendung nach einem der Ansprüche 1 bis 3, wobei die tägliche Dosis an Interleukin-2 Polypeptid als eine einzelne Injektion oder in zwei Injektionen verabreicht wird.

5. Die Verwendung nach einem der vorhergehenden Ansprüche, wobei der synthetische γδT-Aktivator ein Ligand des T-Rezeptors von γδT-Lymphozyten ist.

6. Die Verwendung nach Anspruch 5, wobei der synthetische γδT-Aktivator ausgewählt ist aus Phosphohalohydrin-Verbindungen, Phosphoepoxid-Verbindungen, Biphosphonat-Verbindungen und Alkylaminen.

7. Die Verwendung nach Anspruch 6, wobei der synthetische γδT-Aktivator ausgewählt ist aus den folgenden Verbindungen:
3-(Brommehtyl)-3-Butanol-1-yl-Diphosphat (BrHPP)
3-(Jodmethyl)-3-Butanol-1-yl-Diphosphat (IHPP)
3-(Chlormethyl)-3-Butanol-1-yl-Diphosphat (CIHPP)
3-(Brommehtyl)-3-Butanol-1-yl-Triphosphat (BrHPPP)
3-(Jodmehtyl)-3-Butanol-1-yl-Triphosphat (IHPPP)
α,γ-Di-[3-(Brommethyl)-3-Butanol-1-yl]-Triphosphat (diBrHTP)
α,γ-Di-[3-(Jodmethyl)-3-Butanol-1-yl]-Triphosphat (diIHTP)
3,4-Epoxy-3-Methyl-1-Butyl-Diphosphat (Epox-PP)
3,4-Epoxy-3-Methyl-1-Butyl-Triphosphat (Epox-PPP)
α,γ-Di-3,4-Epoxy-3-Methyl-1-Butyl-Triphosphat (di-Epox-TP)

8. Die Verwendung nach einem der vorhergehenden Ansprüche, wobei der synthetische γδT-Aktivator eine PED oder PHD Verbindung ist und als eine einzelne Injektion in einer Dosis umfassend zwischen 10 und 50 mg/kg am Anfang der Behandlung verabreicht wird, und wobei das Interleukin-2 Polypeptid in einer täglichen Dosis umfassend zwischen 0,2 und 2 ME/m² pro Tag über einen Zeitraum umfassend zwischen 1 und 10 Tagen verabreicht wird.

9. Die Verwendung nach einem der vorhergehenden Ansprüche zur Behandlung von Krebs in einem Subjekt.

10. Die Verwendung nach einem der Ansprüche 1 bis 8 zur Behandlung einer infektiösen Erkrankung in einem Subjekt.

11. Die Verwendung nach einem der Ansprüche 1 bis 8 zur Behandlung einer Autoimmunerkrankung in einem Subjekt.

12. Die Verwendung nach einem der Ansprüche 1 bis 8 zur Behandlung einer Erkrankung, die durch pathologische Zellen verursacht wird oder mit pathologischen Zellen assoziiert ist, die in einem Subjekt empfindlich für die Lyse durch γδT-Zellen sind.

13. Produkt umfassend einen synthetischen γδT-Aktivator und ein Interleukin-2 Polypeptid, für eine getrennte Verwendung zur Behandlung von Krebs, einer infektiösen Erkrankung, einer Autoimmunerkrankung oder einer allergischen Erkrankung in einem humanen Subjekt, wobei der synthetische γδT-Aktivator als eine einzelne Dosis am Anfang der Behandlung in einer Dosis umfassend zwischen 2 und 60 mg/kg und das Interleukin-2 Polypeptid in niedriger Dosis verabreicht wird.

## Revendications

1. Utilisation d'un activateur synthétique de γδT et d'un polypeptide d'interleukine 2 pour la fabrication d'une composition pharmaceutique destinée au traitement d'un cancer, d'une maladie infectieuse, d'une maladie auto-immune ou d'une maladie allergique chez un sujet humain, l'activateur synthétique de γδT et le polypeptide d'interleukine 2 étant administrés séparément au sujet, ledit activateur synthétique de γδT étant administré en un dose unique au début du traitement à une dose comprise entre 2 et 60 mg/kg et ledit polypeptide d'interleukine 2 étant administré à faibles doses.

2. Utilisation selon la revendication 1, dans laquelle le polypeptide d'interleukine 2 est administré sur une période de temps comprise entre 1 et 10 jours.

3. Utilisation selon la revendication 2, dans laquelle le polypeptide d'interleukine 2 est administré à une dose journalière comprise entre 0,2 et 2 MU/m² par jour, de préférence entre 0,2 et 1,5 MU/m², de manière encore plus préférée entre 0,2 et MU/m².

4. Utilisation selon l'une quelconque des revendications 1 à 3, dans laquelle la dose journalière du polypeptide d'interleukine 2 est administrée en une unique injection ou en deux injections.

5. Utilisation selon l'une quelconque des revendications précédentes, dans laquelle l'activateur synthétique de γδT est un ligand du récepteur T des lymphocytes γδT.

6. Utilisation selon la revendication 5, dans laquelle l'activateur synthétique de γδT est sélectionné parmi des composés phosphohalohydrines, des composés phosphoépoxides, des composés biphosphonates et des alkylamines.

7. Utilisation selon la revendication 6, dans laquelle l'activateur synthétique de γδT est sélectionné parmi les composés suivants :
3-(bromométhyl)-3-butanol-1-yl-diphosphate (BrHPP)
3-(iodométhyl)-3-butanol-1-yl-diphosphate (IHPP)
3-(chlorométhyl)-3-butanol-1-yl-diphosphate (CIHPP)
3-(bromométhyl)-3-butanol-1-yl-triphosphate (BrHPPP)
3-(iodométhyl)-3-butanol-1-yl-triphosphate (IHPPP)
α,γ-di-[3-(bromométhyl)-3-butanol-1-yl]-triphosphate (diBrHTP)
α,γ-di-[3-(iodométhyl)-3-butanol-1-yl]-triphosphate (diIHTP)
3,4,-époxy-3-méthyl-1-butyl-diphosphate (Epox-PP)
3,4,-époxy-3-méthyl-1-butyl-triphosphate (Epox-PPP)
α,γ-di-3,4,-époxy-3-méthyl-1-butyl-triphosphate (di-Epox-TP)

8. Utilisation selon l'une quelconque des revendications précédentes, dans laquelle l'activateur synthétique de γδT est un composé PED ou PHD et est administré en une unique injection à une dose comprise entre 10 et 50 mg/kg au début du traitement et dans laquelle le polypeptide d'interleukine 2 est administrée à une dose journalière comprise entre 0,2 et 2 MU/m² par jour sur une période de temps comprise entre 1 et 10 jours.

9. Utilisation selon l'une quelconque des revendications précédentes pour traiter un cancer chez un sujet.

10. Utilisation selon l'une quelconque des revendications 1 à 8 pour traiter une maladie infectieuse chez un sujet.

11. Utilisation selon l'une quelconque des revendications 1 à 8 pour traiter une maladie auto-immune chez un sujet.

12. Utilisation selon l'une quelconque des revendications 1 à 8 pour traiter une maladie provoquée par ou associée à des cellules pathologiques sensibles à une lyse par des cellules γδT chez un sujet.

13. Produit comprenant un activateur synthétique de γδT et un polypeptide d'interleukine 2, pour une utilisation séparée, destiné au traitement d'un cancer, d'une maladie infectieuse, d'une maladie auto-immune ou d'une maladie allergique chez un sujet humain, dans lequel ledit activateur synthétique de γδT est administré en un dose unique au début du traitement à une dose comprise entre 2 et 60 mg/kg et ledit polypeptide d'interleukine 2 est administré à faibles doses.
